# EUROPEAN PATENT APPLICATION

(11) **EP 4 296 182 A1**
(43) Date of publication of application: **27.12.2023**
(21) Application number: 23174600.9
(22) Date of filing: 22.05.2023
(51) Int. Cl.: B65D 21/02, A61M 5/00, B65D 25/10

(54) **FRAME FOR HOLDING SECONDARY PHARMACEUTICAL PACKAGING**

(30) Priority: 21.06.2022 EP 22180111
(71) Applicant: SCHOTT Pharma Schweiz AG, 9000 St. Gallen (CH)
(72) Inventor: WOLF, Patrick, 9001 St. Gallen (CH); KLOKE, Arne, 9001 St. Gallen (CH); SCHNETZER, Joel, 9001 St. Gallen (CH); VASANTHAMOHAN, Nithan, 9001 St. Gallen (CH); DURAKOVIC, Belim, 9001 St. Gallen (CH)
(74) Representative: Schott Corporate IP

(57) **Abstract**

The invention relates to a special a frame for holding secondary pharmaceutical packaging, a method for handling the special frames, a kit comprising the special frames and a use of the special frame.

## Description

Described herein is a frame for holding secondary pharmaceutical packaging, a method for handling frames, a kit of frames and a use of a frame.

After manufacturing pharmaceutical compounds are usually filled and distributed in containers, so-called primary pharmaceutical packaging. This usually involves uncleaned primary pharmaceutical packaging, which needs to be cleaned and sterilized before subsequent filling and sealing. In order to simplify and provide flexibility to this complex process for manufactures of pharmaceutical compounds, pre-cleaned and sterilized primary pharmaceutical packaging are offered in nests or tubs with nests, so-called secondary pharmaceutical packaging. The primary pharmaceutical packaging can be stored in the secondary pharmaceutical packaging. The secondary pharmaceutical packaging can at least in part or fully be enclosed by at least one bag or pouch. These so-called ready-to-use packaging systems may be directly unpacked, filled and resealed by the pharmaceutical company under controlled sterile conditions.

For transport to the pharmaceutical manufacturer, the ready-to-use packaging systems are stacked loosely in a transport box. One problem encountered in the case of these ready-to-use packaging systems is that further automation is hindered. The secondary pharmaceutical packaging has to be removed from the transport box for further processing. The secondary pharmaceutical packaging has to be placed in a different box or holding device for fill and finish, e.g. sealing or packaging, of the primary pharmaceutical packaging.

A further problem that occurs for state of the art ready-to-use packaging systems is that the transport box is disposed after transport to the pharmaceutical manufacturer. The transport box is not suitable for further processing. Thus, for state of the art transport boxes and processes, waste is increasing and reuse over multiple production cycles including the return of secondary pharmaceutical packaging to the manufacturer of primary pharmaceutical packaging is not possible.

A further problem that occurs is that state of the art transport boxes easily deform. In fact, during storage, transport or further processing the transport box changes its shape. This leads to transport damage, e.g. mechanical impact or wear due to protruding, or faults during handling, e.g. jamming.

These problems were extensively investigated and the inventors have found that, surprisingly, a lack of automation, reuse and reliability is caused by the handling of secondary pharmaceutical packaging and may be increased by a special frame.

It is thus an object of the present invention to provide a frame for holding secondary pharmaceutical packaging, a method for handling frames, a kit of frames and a use of a frame which overcome the above-described problems and which enable further automation of processing primary pharmaceutical packaging, the reuse of secondary pharmaceutical packaging and/or more reliable storage, transport and further processing.

This object is achieved by a frame for holding secondary pharmaceutical packaging comprising, a base plate, at least two sidewalls connected to the base plate, at least one stacking element located on the base plate, wherein the base plate comprises at least two openings, wherein the openings are formed to hold the secondary pharmaceutical packaging.

The object is likewise achieved by a method for handling frames comprising the following steps, preferably in this order:
i) providing a first frame described herein,
ii) placing at least one secondary pharmaceutical packaging in the first frame,
iii) providing a second frame described herein,
iv) placing at least one secondary pharmaceutical packaging in the second frame,
v) stacking the second frame on top of the first frame.

The object is likewise achieved by a kit of frames comprising, at least two frames described herein, preferably wherein the frames are stacked on top of each other.

The object is likewise achieved by a use of a frame described herein to distribute or store secondary and preferably primary pharmaceutical packaging.

In the present application, all singular terms shall also include the plural and all plural terms shall also include the singular unless otherwise stated. For example, all limitations and preferred embodiments of a primary/secondary pharmaceutical packaging shall, in particular, also apply to a plurality of, for example, 2 or more primary/secondary pharmaceutical packaging. Furthermore, all limitations and preferred embodiments of the frame shall also apply to the method, kit and use and vice versa unless otherwise stated.

### Frame

The frame according to the invention is a frame for holding secondary pharmaceutical packaging comprising, a base plate, at least two sidewalls connected to the base plate, at least one stacking element located on the base plate, wherein the base plate comprises at least two openings, wherein the openings are formed to hold the secondary pharmaceutical packaging. The frame facilitates further automation, such as filling and finishing, e.g. sealing or packaging, primary pharmaceutical packaging without removal of the secondary pharmaceutical packaging. Further, the frame is robust and flexible and can thus be reused over multiple production cycles or to return primary and/or secondary pharmaceutical packaging for recycling or reuse.

The term "secondary pharmaceutical packaging" herein is to be understood as a nest or a tub comprising a nest, wherein the nest is formed to hold primary pharmaceutical packaging. Preferably, the tub is a tub according to ISO 11040-7:2014(E). Preferably, the nest is a nest selected from the commercially available SCHOTT iQ^{®} platform.

The term "nest" herein refers to an article for holding the primary pharmaceutical packaging. Thus, all primary pharmaceutical packaging are in direct contact with the nest. The nest preferably comprises 10 to 200, preferably 16 to 160, more preferably 40 to 100, primary pharmaceutical packaging.

The term "primary pharmaceutical packaging" herein is to be understood as a vial, cartridge, syringe or an ampoule. Primary pharmaceutical packaging is capable of receiving and storing pharmaceutical compounds.

In a preferred embodiment the stacking element is formed to hold a further, preferably identical, frame in place.

The stacking elements enable space saving and hold the frames reliable in place due to the restricted movement. Further, this increases the stability of a stack of the frames.

In a preferred embodiment the frame comprises three, preferably four sidewalls.

An increased number of sidewalls leads to a higher stability and improved stacking of the frame. Further, mechanical impact on the held secondary pharmaceutical packaging can be reduced. Further, light sensitive pharmaceutical compounds can be processed, as the sidewalls serve as a light shield. Further, contamination can be reduced, as the sidewalls shield against foreign particles. Further, an increased surface area on the side of the frame enables covering a frame with wrapping foil.

In a preferred embodiment the frame comprises 3 to 15, preferably 4 to 14, more preferably 5 to 13, even more preferably 6 to 12, most preferably 8 to 10, openings.

It is advantageous to enable the accommodation of state of the art secondary pharmaceutical packaging in the frame. Further, the weight of the frame can be reduced. Further, the placement and removal of the secondary pharmaceutical packaging can be automatized.

In a preferred embodiment the secondary pharmaceutical packaging is a nest or a tub with a nest,
preferably wherein the tub is a tub according to ISO 11040-7:2014(E),
preferably wherein the nest is formed to hold primary pharmaceutical packaging,
more preferably wherein primary pharmaceutical packaging is a vial, cartridge, syringe or an ampoule.

In a preferred embodiment the frame comprises 2, preferably4, more preferably 6, even more preferably 8, most preferably 10, stacking elements.

An increased number of stacking elements ensures to hold the frame in place during stacking and facilitate improved processing and transport. However, too many stacking elements complicate handling of the frames, e.g. canting, and add to the weight of the frame.

In a preferred embodiment the base plate comprises at least two, preferably four, edges, preferably wherein the two, preferably four, sidewalls are located respectively at an edge, and/or
the base plate comprises at least one, preferably four, corner(s).

It is advantageous to use state of the art pallets, e.g. wood pallets, for transport of the frame. Further, transport damage can be reduced.

The frame may consist of any material. In a preferred embodiment the base plate, the sidewalls and/or the stacking elements consist of the same material.

It is advantageous to use the same material to facilitate reuse and recycling.

In a preferred embodiment the base plate, the sidewalls and/or the stacking elements are connected by a material fit or made from one piece.

It is advantageous to firmly connect the elements of the frame facilitate reuse and recycling.

In a preferred embodiment the base plate comprises an upper surface and a lower surface and/or
the base plate comprises notches enclosing each opening,
preferably wherein a notch has a width of 8 mm to 16 mm, preferably 10 mm to 12 mm, and/or a depth of 1 mm to 2 mm, preferably 1.4 mm to 1.6 mm.

It is advantageous to enable the accommodation of state of the art secondary pharmaceutical packaging in the frame.

The length, width and height of the frame is not particularly restricted. In a preferred embodiment the frame has a width of 200 mm to 1000mm, preferably 240 mm to 800 mm, a length of 600 mm to 1400 mm, preferably 800 mm to 1200 mm, and/or a height of 80 mm to 104 mm, preferably 84 mm to 102 mm.

It is advantageous to use state of the art pallets, e.g. wood pallets, for transport of the frame. Further, transport damage can be reduced.

The length and width of an opening is not particularly restricted. In a preferred embodiment, an opening has a width of 160 mm to 240 mm, preferably 190 mm to 210 mm, more preferably 202 mm to 208 mm, and/or a length of 190 mm to 270 mm, preferably 220 mm to 250 mm, more preferably 235 mm to 240 mm.

It is advantageous to enable the accommodation of state of the art secondary pharmaceutical packaging in the frame.

In a preferred embodiment the sidewalls comprise at least one, preferably 2, more preferably 4, holes or cutouts, preferably wherein the holes or cutouts are formed as finger grips.

The holes or cutouts can increase the degree of automation, such that machines or workers can be reliably hold and processes the frames.

In a preferred embodiment the frame comprises, preferably consists of, a polymer, more preferably comprises HDPE, PP or ABS, and/or
the frame consist of an intransparent, preferably intransparent for visible light and/or UV light, material.

Durable and robust materials allow for reuse of the frames, without breakage or particle generation. Further, the material is lightweight and recyclable. It is advantageous to use intransparent materials for the frame in order to process light sensitive pharmaceutical compounds.

In a preferred embodiment the frame has a rectangular shape and/or
the openings have a rectangular shape, preferably with rounded edges.

It is advantageous to use state of the art pallets, e.g. wood pallets, for transport of the frame. Further, transport damage can be reduced.

In a preferred embodiment the stacking element is in form of a notch, preferably embedded in the base plate and more preferably located at an edge of the base plate and/or
the stacking element is in form of a protrusion, preferably located at an edge of the base plate and/or
the stacking element is in form of a thorn, preferably located at a corner of the base plate.

Robust stacking elements ensure to hold the frame in place during stacking and facilitate improved processing and transport. Further, visual inspection of the stacking can be simplified, e.g. spaces between stacked frames. Further, the dimensional variance of a stack of frames is decreased.

In a preferred embodiment the base plate comprises an upper surface defining a first surface area and
the at least two sidewalls comprise each a lower edge defining a second surface area, wherein the ratio of the first surface area to the second surface area is 0.9 to 1.1, preferably 0.95 to 1.05, more preferably 0.97 to 1.03, even more preferably 0.98 to 1.02, most preferably 0.99 to 1.01.

An optimised ratio between the first (upper) and second (lower) surface are facilitates reliable stacking of the frame. An increased amount of room to move leads to wear of the frames and decreases the stability of a stack. However, a very small clearance causes faults during automated processing, e.g. canting. Further, the dimensional variance of a stack of frames is decreased.

In a preferred embodiment the openings are arranged equidistant with respect to the at least one of the two, preferably both, sidewalls or
the openings are staggered.

It is advantageous to enable the accommodation of state of the art secondary pharmaceutical packaging in the frame and to increase the accessibility with a defined pattern. Further, the packing density is increased.

In a preferred embodiment the base plate comprises an upper surface,
wherein the sidewalls comprise an upper edge and
wherein the upper edge is flush with the upper surface.

It is advantageous to avoid unnecessary protrusions, which could cause wear on the frames, e.g. generate particles, or lead to faults during automated processing due to canting. Further, the stability of a stack of frames is increased.

### Method

The method according to the invention is a method for handling frames comprising the following steps, preferably in this order:
i) providing a first frame described herein,
ii) placing at least one secondary pharmaceutical packaging in the first frame,
iii) providing a second frame described herein,
iv) placing at least one secondary pharmaceutical packaging in the second frame,
v) stacking the second frame on top of the first frame.

In a preferred embodiment the method further comprises the step(s):
placing at least one primary pharmaceutical packaging in a secondary pharmaceutical packaging, and
preferably filling the primary pharmaceutical packaging, preferably with a pharmaceutical substance and/or
preferably inspecting the primary pharmaceutical packaging.

In a preferred embodiment the method further comprises the step(s):
sealing and/or enclosing the secondary pharmaceutical packaging, preferably with a pouch and/or at least one, preferably two, bag(s),
preferably before placing the secondary pharmaceutical packaging in the first and/or second frame, and
preferably removing a pouch and/or at least one, preferably two, bag(s) from the secondary pharmaceutical packaging.

In a preferred embodiment the method further comprises the step:
removing the secondary pharmaceutical packaging from the first and/or second frame.

In a preferred embodiment the method further comprises the step:
placing a cover plate on top of the second frame.

In a preferred embodiment the method further comprises the step
stacking further frames described herein on top of the second frame and/or below the first frame.

### Kit

The kit according to the invention is a kit of frames comprising,
at least two frames described herein,
preferably wherein the frames are stacked on top of each other.

The kit facilitates further automation, such as filling and finishing, e.g. sealing or packaging, primary pharmaceutical packaging without removal of the secondary pharmaceutical packaging. Further, the kit enables the reuse of frames over multiple production cycles or the return of primary and/or secondary pharmaceutical packaging for recycling or reuse.

In a preferred embodiment the kit further comprises at least one secondary pharmaceutical packaging,
preferably held by a frame, and
preferably further comprising primary pharmaceutical packaging,
preferably stored in the secondary pharmaceutical packaging, and
more preferably wherein the secondary pharmaceutical packaging is sealed by a pouch and/or enclosed by one, preferably two, bag(s).

In a preferred embodiment the kit comprises 4 to 80, preferably 10 to 50, more preferably 20 to 40, most preferably 25 to 30, frames.

It is advantageous to use state of the art pallets, e.g. wood pallets, for transport of the frame. Further, transport damage can be reduced. Further, it is advantageous to enable the accommodation of state of the art secondary pharmaceutical packaging in the frame.

The length, width and height of the kit is not particularly restricted. In a preferred embodiment the kit has a width of 600 mm to 1000mm, preferably about 800 mm, a length of 1000 mm to 1400 mm, preferably about 1200 mm, and/or a height of 1400 mm to 1800 mm, preferably about 1600 mm.

It is advantageous to use state of the art pallets, e.g. wood pallets, for transport of the frame. Further, transport damage can be reduced. Further, it is advantageous to enable the accommodation of state of the art secondary pharmaceutical packaging in the frame.

In a preferred embodiment the kit further comprises at least one cover plate,
preferably wherein the cover plate is formed to be placed on the base plate,
more preferably further comprising wrapping foil, preferably enclosing the frames and the cover plate.

The cover plate can further protect the primary and/or secondary pharmaceutical packaging. Additionally, the cover plate can enable processing of light sensitive pharmaceutical compounds.

In a preferred embodiment the frames are arranged in 1 to 5 stacks, preferably 2 to 4 stacks, more preferably 3 and
preferably wherein one stack consists of 8 to 20, preferably 10 to 16, more preferably 12 to 14, frames.

It is advantageous to use state of the art pallets, e.g. wood pallets, for transport of the frame. Further, transport damage can be reduced. Further, it is advantageous to enable the accommodation of state of the art secondary pharmaceutical packaging in the frame.

In a preferred embodiment the kit comprises at least one pouch,
wherein the pouch covers an opening,
preferably wherein the pouch is connected to the base plate.

It is advantageous to seal the secondary packing with the aid of the base plate. This eases further processing, such as unpacking pre-sterilized primary and/or secondary pharmaceutical packaging. Further, sealing on an increased area of a flat surface is more reliable and further bags or pouches can be omitted.

### Use

The use according to the invention is a use of a frame described herein to distribute or store secondary and preferably primary pharmaceutical packaging.

### Figures

- Figure 1:: Spatial view of a frame according to an embodiment of the invention
- Figure 2:: Top view of a frame according to an embodiment of the invention
- Figure 3:: Side view of a kit of frames according to an embodiment of the invention

### List of reference numerals

- 1: Frame
- 2: Base plate
- 3: Sidewalls
- 4: Stacking element
- 5: Openings
- 6: Secondary pharmaceutical packaging
- 7: Kit of frames

### Detailed description of the figures

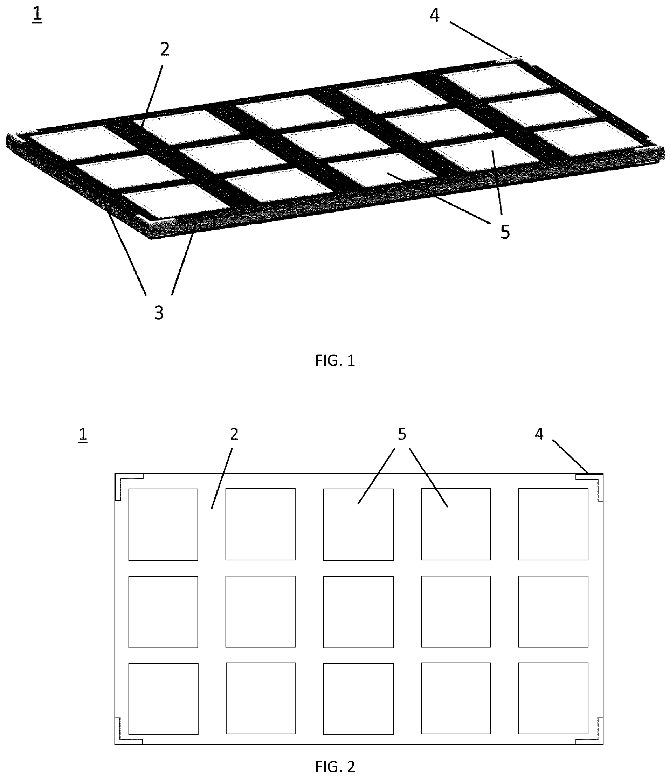

Figure 1 shows a spatial view of a frame (1) according to an embodiment of the invention and Fig. 2 shows a top view of a frame (1) according to an embodiment of the invention. As can be seen from Figures 1 and 2 the frame (1) comprises, a base plate (2), at least two sidewalls (3) (not shown in Fig. 2) connected to the base plate (2), at least one stacking element (4) located on the base plate (2). The base plate (2) comprises at least two openings (5) and the openings (5) are formed to hold the secondary pharmaceutical packaging (6) (not shown).

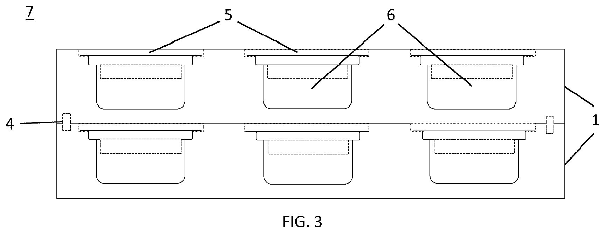

Figure 3 shows a cross-sectional side view of a kit of frames (7) according to an embodiment of the invention. As can be seen from Fig. 3 the kit comprises at least two frames (1) described herein, for example as depicted in Figure 1 and 2, wherein the frames (1) are stacked on top of each other. The kit comprises secondary pharmaceutical packaging (7), for example a tub comprising a nest. The stacking elements (4) hold the respective frame in place.

## Claims

1. Frame (1) for holding secondary pharmaceutical packaging (6) comprising,
a base plate (2),
at least two sidewalls (3) connected to the base plate (2),
at least one stacking element (4) located on the base plate (2),
wherein the base plate (2) comprises at least two openings (5),
wherein the openings (5) are formed to hold the secondary pharmaceutical packaging (6).

2. The frame (1) according to claim 1,
wherein the stacking element (4) is formed to hold a further, preferably identical, frame (1) in place.

3. The frame (1) according to claim 1 or 2,
comprising three, preferably four sidewalls (3).

4. The frame (1) according to any one of the preceding claims,
comprising 3 to 15, preferably 4 to 14, more preferably 5 to 13, even more preferably 6 to 12, most preferably 8 to 10, openings (5).

5. The frame (1) according to any one of the preceding claims,
wherein the secondary pharmaceutical packaging (6) is a nest or a tub with a nest, preferably wherein the tub is a tub according to ISO 11040-7:2014(E),
preferably wherein the nest is formed to hold primary pharmaceutical packaging, more preferably wherein primary pharmaceutical packaging is a vial, cartridge, syringe or an ampoule.

6. The frame (1) according to any one of the preceding claims,
comprising 2, preferably 4, more preferably 6, even more preferably 8, most preferably 10, stacking elements (4).

7. The frame (1) according to any one of the preceding claims,
wherein the base plate (2) comprises at least two, preferably four, edges,
preferably wherein the two, preferably four, sidewalls (3) are located respectively at an edge, and/or
wherein the base plate (2) comprises at least one, preferably four, corner(s).

8. The frame (1) according to any one of the preceding claims,
wherein the base plate (2), the sidewalls (3) and/or the stacking elements (4) consist of the same material.

9. The frame (1) according to any one of the preceding claims,
wherein the base plate (2), the sidewalls (3) and/or the stacking elements (4) are connected by a material fit or made from one piece.

10. The frame (1) according to any one of the preceding claims,
wherein the base plate (2) comprises an upper surface and a lower surface and/or
wherein the base plate (2) comprises notches enclosing each opening (5),
preferably wherein a notch has a width of 8 mm to 16 mm, preferably 10 mm to 12 mm, and/or a depth of 1 mm to 2 mm, preferably 1.4 mm to 1.6 mm.

11. The frame (1) according to any one of the preceding claims,
wherein the frame (1) has a width of 200 mm to 1000mm, preferably 240 mm to 800 mm, a length of 600 mm to 1400 mm, preferably 800 mm to 1200 mm, and/or a height of 80 mm to 104 mm, preferably 84 mm to 102 mm.

12. The frame (1) according to any one of the preceding claims,
wherein an opening (5) has a width of 160 mm to 240 mm, preferably 190 mm to 210 mm, more preferably 202 mm to 208 mm, and/or a length of 190 mm to 270 mm, preferably 220 mm to 250 mm, more preferably 235 mm to 240 mm.

13. The frame (1) according to any one of the preceding claims,
wherein the sidewalls (3) comprise at least one, preferably 2, more preferably 4, holes or cutouts, preferably wherein the holes or cutouts are formed as finger grips.

14. The frame (1) according to any one of the preceding claims,
wherein the frame (1) comprises, preferably consists of, a polymer, more preferably comprises HDPE, PP or ABS, and/or
wherein the frame (1) consist of an intransparent, preferably intransparent for visible light and/or UV light, material.

15. The frame (1) according to any one of the preceding claims,
wherein the frame (1) has a rectangular shape and/or
the openings (5) have a rectangular shape, preferably with rounded edges.

16. The frame (1) according to any one of the preceding claims,
wherein the stacking element (4) is in form of a notch, preferably embedded in the base plate (2) and more preferably located at an edge of the base plate (2) and/or
wherein the stacking element (4) is in form of a protrusion, preferably located at an edge of the base plate (2) and/or
wherein the stacking element (4) is in form of a thorn, preferably located at a corner of the base plate (2).

17. The frame (1) according to any one of the preceding claims,
wherein the base plate (2) comprises an upper surface defining a first surface area and wherein the at least two sidewalls (3) comprise each a lower edge defining a second surface area,
wherein the ratio of the first surface area to the second surface area is 0.9 to 1.1, preferably 0.95 to 1.05, more preferably 0.97 to 1.03, even more preferably 0.98 to 1.02, most preferably 0.99 to 1.01.

18. The frame (1) according to any one of the preceding claims,
wherein the openings (5) are arranged equidistant with respect to the at least one of the two, preferably both, sidewalls (3) or
wherein the openings (5) are staggered.

19. The frame (1) according to any one of the preceding claims,
wherein the base plate (2) comprises an upper surface,
wherein the sidewalls (3) comprise an upper edge and
wherein the upper edge is flush with the upper surface.

20. Method for handling frames (1) comprising the following steps, preferably in this order:
i) providing a first frame (1) according to any one of the preceding claims,
ii) placing at least one secondary pharmaceutical packaging (6) in the first frame (1),
iii) providing a second frame (1) according to any one of the preceding claims,
iv) placing at least one secondary pharmaceutical packaging (6) in the second frame (1),
v) stacking the second frame (1) on top of the first frame (1).

21. The method according to claim 20,
further comprising the step(s):
placing at least one primary pharmaceutical packaging in a secondary pharmaceutical packaging (6), and
preferably filling the primary pharmaceutical packaging, preferably with a pharmaceutical substance and/or
preferably inspecting the primary pharmaceutical packaging.

22. The method according to claims 20 or 21,
further comprising the step(s):
sealing and/or enclosing the secondary pharmaceutical packaging (6), preferably with a pouch and/or at least one, preferably two, bag(s),
preferably before placing the secondary pharmaceutical packaging (6) in the first and/or second frame (1), and
preferably removing a pouch and/or at least one, preferably two, bag(s) from the secondary pharmaceutical packaging (6).

23. The method according to any one of claims 20 to 22,
further comprising the step:
removing the secondary pharmaceutical packaging (6) from the first and/or second frame (1).

24. The method according to any one of claims 20 to 23,
further comprising the step:
placing a cover plate on top of the second frame (1).

25. The method according to any one of claims 20 to 24,
further comprising the step:
stacking further frames (1) according to one of claims 1 to 19 on top of the second frame (1) and/or below the first frame (1).

26. Kit of frames (7) comprising,
at least two frames (1) according to any one of claims 1 to 19,
preferably wherein the frames (1) are stacked on top of each other.

27. The kit (7) according to claim 26,
further comprising at least one secondary pharmaceutical packaging (6),
preferably held by a frame (1), and
preferably further comprising primary pharmaceutical packaging,
preferably stored in the secondary pharmaceutical packaging (6), and
more preferably wherein the secondary pharmaceutical packaging (6) is sealed by a pouch and/or enclosed by one, preferably two, bag(s).

28. The kit (7) according to claims 26 or 27,
comprising 4 to 80, preferably 10 to 50, more preferably 20 to 40, most preferably 25 to 30, frames (1).

29. The kit (7) according to any one of claims 26 to 28,
wherein the kit (7) has a width of 600 mm to 1000mm, preferably about 800 mm, a length of 1000 mm to 1400 mm, preferably about 1200 mm, and/or a height of 1400 mm to 1800 mm, preferably about 1600 mm.

30. The kit (7) according to any one of claims 26 to 29,
further comprising at least one cover plate,
preferably wherein the cover plate is formed to be placed on the base plate (2),
more preferably further comprising wrapping foil, preferably enclosing the frames (1) and the cover plate.

31. The kit (7) according to any one of claims 26 to 30,
wherein the frames (1) are arranged in 1 to 5 stacks, preferably 2 to 4 stacks, more preferably 3 and
preferably wherein one stack consists of 8 to 20, preferably 10 to 16, more preferably 12 to 14, frames (1).

32. The kit (7) according to any one of claims 26 to 31,
comprising at least one pouch,
wherein the pouch covers an opening (5),
preferably wherein the pouch is connected to the base plate (2).

33. Use of a frame (1) according to any one of claims 1 to 19 to distribute or store secondary and preferably primary pharmaceutical packaging.
